# FASCICULE DE BREVET EUROPEEN

(11) **EP 0 605 276 B1**
(45) Date de publication et mention de la délivrance du brevet: **02.02.2000**
(21) Numéro de dépôt: 93403042.0
(22) Date de dépôt: 15.12.1993
(51) Int. Cl.: A61F 2/02

(54) **Dispositif pouvant constituer sélectivement un filtre sanguin temporaire**
Vorrichtung zur mögliche erwählte Verwendung als zeitweiliger Blutgefässfilter
Device for selective use as a temporary blood-filter

(30) Priorité: 28.12.1992 FR 9215774
(43) Date de publication de la demande: 06.07.1994
(73) Titulaire: B. BRAUN CELSA, 92100 Boulogne-Billancourt (FR)
(72) Inventeur: Nadal, Guy, F-86000 Poitiers (FR)
(74) Mandataire: Lerner, François

(56) Documents cités:
- EP-A- 0 348 295
- EP-A- 0 423 916
- EP-A- 0 466 518
- WO-A-92/19310
- DE-A- 4 030 998
- DE-U- 8 812 719
- FR-A- 2 573 646
- FR-A- 2 657 261
- US-A- 4 650 466

## Description

L'invention se rapporte aux dispositifs propres à être implantés à l'intérieur d'un vaisseau pour y constituer un filtre sanguin pouvant être temporaire.

De tels filtres sont souvent destinés à être installés dans la veine cave, telle que la veine cave inférieure, ceci en particulier par voie percutanée.

On sait depuis déjà des années que les filtres laissés à titre définitif peuvent entraîner, à long terme, des complications non négligeables pour le patient, telles que des obstructions de vaisseau et des séquelles veineuses.

Ces complications sont aujourd'hui acceptées en particulier lorsqu'on est en présence d'un risque d'embolie pulmonaire constant au cours du temps.

Il est néanmoins apparu souhaitable de pouvoir retirer un tel "barrage", à chaque fois notamment que le risque de récidive de l'embolie ne crée plus de danger pour le malade.

Les filtres temporaires existants ont cette faculté de pouvoir être mis en place pour assurer une protection uniquement pendant le temps nécessaire, puis d'être retirés ensuite.

Parmi les filtres connus, on peut citer ceux décrits dans FR-A-2 657 261 et FR-A-2 689 388.

Dans EP-A-423 961 est par ailleurs décrit un dispositif propre à être implanté à l'intérieur d'un vaisseau du corps d'un patient, et comprenant :
- une structure comprenant une série de pattes allongées essentiellement sensiblement parallèlement à un axe pour définir, suivant cet axe, une surface tubulaire ouverte à travers laquelle le sang peut passer, cette structure présentant un premier diamètre de tube réduit, pour son implantation jusqu'au vaisseau, et un second diamètre de tube expansé, pour être propre à venir au contact de ce vaisseau dans une position implantée à l'intérieur de celui-ci,
- et des moyens de sollicitation des pattes de la structure, alors que celle-ci présente son second diamètre expansé de tube, pour créer une zone de striction.

Il ne s'agit toutefois pas là d'un filtre sanguin mais d'un "écarteur", ou "élargisseur de vaisseau", également dénommé "stent'.

En outre, le problème posé dans ce document consiste à assurer un retrait possible de l'écarteur avec possibilité de l'associer à d'autres écarteurs, bout à bout, sans nécessiter de multiples implantations successives.

Dans l'invention, le problème posé est différent puisqu'il consiste en ce qu'une structure telle que décrite ci-avant puisse constituer sélectivement un filtre sanguin temporaire.

Ainsi, il n'existe pas aujourd'hui, à la connaissance du demandeur, de matériel qui permette de filtrer le sang "in situ" pendant une durée donnée, supérieure éventuellement à plusieurs mois, sans que le patient ait à supporter les complications à long terme des filtres définitifs actuels pouvant être laissés en place à vie.

Pour résoudre ce problème, ii est prévu dans l'invention que le dispositif présenté ci-avant soit tel que, pour constituer un filtre sanguin temporaire sélectif, les moyens de sollicitation sont reliés à certaines seulement des pattes afin
soit de rapprocher de l'axe les pattes sollicitées jusqu'à une position de filtration appropriée à la retenue de caillots pouvant être contenus dans le sang passant à travers la structure,
soit, à partir de cette position de filtration, d'écarter radialement dudit axe ces mêmes pattes, en faisant alors varier la capacité de filtration du dispositif.

De cette manière, on va obtenir un filtre à durée de filtration adaptable, tout en faisant évoluer en fonction des besoins le pouvoir filtrant du dispositif, ceci sans avoir nécessairement à retirer ce dernier une fois son rôle de filtre terminé.

Eventuellement même, on pourra envisager de faire jouer au dispositif son rôle de filtre uniquement par périodes.

Etant donné qu'il n'aura plus besoin d'être retiré nécessairement pour cesser de filtrer le sang, le dispositif pourra en conséquence être laissé "à vie", sans que l'on ait à tenir compte des paramètres d'infection ou d'adhérence au cours du temps sur les parois du vaisseau.

Pour une bonne efficacité, le dispositif de l'invention sera, selon une caractéristique complémentaire, avantageusement tel que:
- sa structure présente une série de lignes reliées par des portions d'extrémité courbées, pour définir lesdites pattes, suivant une configuration en zigzags enroulée selon ladite surface tubulaire ouverte,
- et les moyens de sollicitation sont reliés à certaines seulement desdites portions d'extrémité courbées, à l'une des extrémités de la structure. Et en outre, la partie précitée de structure soumise à sollicitation définira avantageusement sensiblement un cône ou un tronc de cône en position rapprochée vers l'axe de la surface de révolution, tandis qu'en position écartée radialement, cette même partie de structure définira de préférence un tronc de cône relativement peu évasé ou un cylindre.

Pour la meilleure sécurité possible, la structure en question sera en outre équipée de moyens d'accrochage, tels que des crochets, pour une fixation à la paroi du vaisseau. Ainsi, on limitera très largement les risques de migration du dispositif.

En tant que moyen de commande à distance des pattes de filtration, on pourra par ailleurs en particulier prévoir l'utilisation d'une fine pince de chirurgien pouvant être glissée à travers le corps du patient jusqu'à la zone d'implantation du dispositif, cette pince pouvant alors être employée pour couper un fil chirurgical formant moyen de retenue en position rapprochée de la partie concernée de structure, laquelle partie, une fois libérée du lien, pourra naturellement s'expanser si die a été prévue en une structure élastiquement déformable à expansion radiale.

En variante, on pourrait envisager l'emploi d'un fin cathéter à travers lequel pourra passer un fil présentant une boucle adaptée pour traverser des passages ménagés dans ladite structure, vers l'endroit à partir duquel la variation de section doit s'opérer. Dans ce cas, l'extrémité proximale de ce cathéter la plus proche de la surface de la peau pourra même être logée en sous cutanée, sans ressortir aucunement du corps du patient entre deux interventions, comme cela est décrit dans les demandes FR-A-2 657 261 ou FR-A-2 697 995.

Si une chambre d'injection implantable est prévue, il sera alors en outre possible de diffuser à l'endroit du filtre un produit traitant, à chaque fois que nécessaire.

Une description plus détaillée de l'invention va maintenant être fournie en référence aux dessins annexés donnés uniquement à titre d'exemples non limitatifs et dans lesquels :
la figure 1 montre une variante préférentielle de réalisation du dispositif de l'invention, illustrée ici en position filtrante,
la figure 2 montre le même dispositif en position non filtrante,
les figures 3 et 4 montrent respectivement le dispositif des figures 1 et 2 en vue de dessus, dans le sens des flèches III et IV des figures 1 et 2,
les figures 5 à 8 montrent différentes étapes d'implantation (figures 5 et 6) ou d'utilisation (figures 7 et 8) du dispositif illustré sur les figures précédentes,
et les figures 9 et 10 montrent chacune une variante de réalisation du moyen de commande à distance de la modification de la section radiale du dispositif.

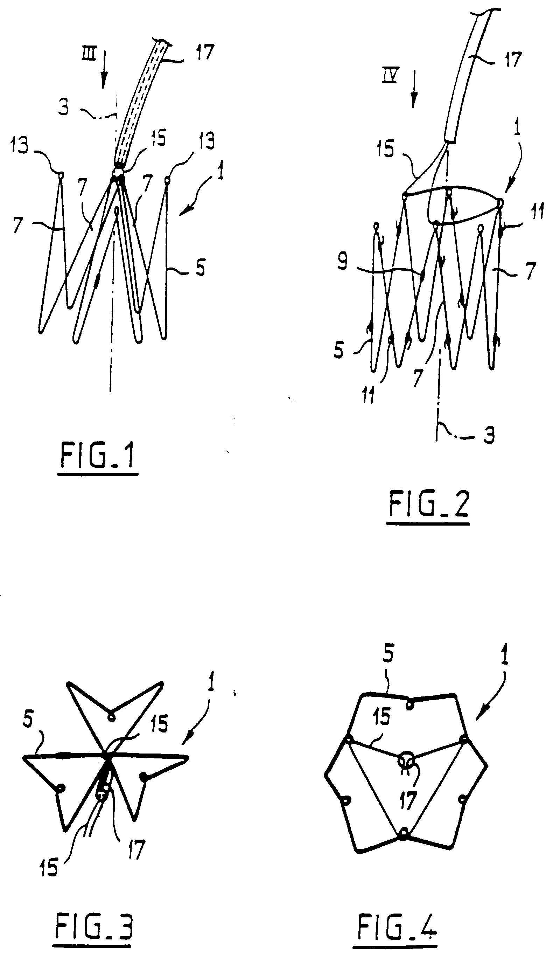

Sur les figures 1 à 4 tout d'abord, on voit donc illustré un dispositif 1 destiné à assurer, au moins temporairement, la fonction d'un filtre sanguin propre à intercepter les éventuels caillots pouvant circuler dans ce sang.

Ce dispositif monobloc est ici réalisé comme une structure auto-expansible radialement à son axe 3 pour, une fois lachée dans le vaisseau choisi, se centrer sensiblement dans l'axe dudit vaisseau.

Comme on peut le constater, la structure 1 est ici constituée d'un fil de préférence rond, relativement rigide 5, conformé en zigzag dans la direction générale de l'axe 3 et enroulé sur lui-même pour présenter une configuration fermée définissant en l'espèce, et dans sa position non contrainte de la figure 2, une surface tubulaire de paroi sensiblement cylindrique (ou légèrement tronconique) adaptée pour venir se plaquer contre la paroi interne du vaisseau choisi, par ces tronçons de pattes en "V", 7.

En 9, on a représenté la zone de fermeture du fil où ses extrémités se rejoignent.

En l'espèce, la paroi tubulaire de la structure 1 est donc constitué d'une série de pattes filiformes allongées essentiellement sensiblement suivant l'axe 3 et adaptées pour laisser passer entre elles le sang mais suffisamment serrées pour retenir les caillots.

De petits crochets 11 d'accrochage à la paroi du vaisseau limiteront en outre avantageusement les risques de migration du dispositif.

En pratique, ces crochets seront toutefois de préférence non prévus sur certaines des pattes 7, étant donné, comme le montre la figure 1, que certaines d'entre elles (par exemple une sur deux) sont prévues pour pouvoir être rapprochées les unes des autres en direction de l'axe 3, en formant alors sensiblement un cône ou un tronc de cône assez fermé, restreignant ainsi localement la section d'une partie au moins du dispositif de telle sorte que ce dernier puisse jouer pleinement son rôle de filtre sanguin.

Pour assurer cette commande en variation de section, on a prévu de conformer en boucle 13 certaines au moins des zones de pliure du fil, ceci à l'une au moins des extrémités axiales de la structure.

Et à l'intérieur du passage défini par une boucle sur deux, on a fait passer ici un fil 15.

En serrant plus ou moins ce lien, on pourra donc passer à volonté de la forme des figures 1 ou 3 à celle des figures 2 ou 4.

Bien entendu, ce lien peut être utilisé seul.

Tandis que la structure en zigzag 1 sera avantageusement réalisée en métal de quelques dixièmes de mm de section, par exemple en acier au cobalt (habituellement dénommée sous la marque déposée "PHYNOX"), le filament 15 pourra être réalisé en une trame bio-compatible sécable, ou éventuellement biodégradable de manière à être alors résorbable au contact du sang.

Dans ce dernier cas, on pourra notamment utiliser de l'acide polyglycolique ou un acide polylactique.

Une autre solution consiste à utiliser un fil chirurgical, non résorbable biologiquement, de le passer en boucle à travers les oeillets 13 choisis et de faire remonter ses deux extrémités opposées à travers un fin cathéter souple 17 qui pourra cheminer tout le long de la voie d'accès reliant le point de ponction ménagé à travers la peau et la zone d'implantation dans le vaisseau, permettant ainsi de commander depuis l'extérieur du corps du patient le resserrement ou le relâchement du lien 15 et donc d'adapter en conséquence le pouvoir filtrant du dispositif.

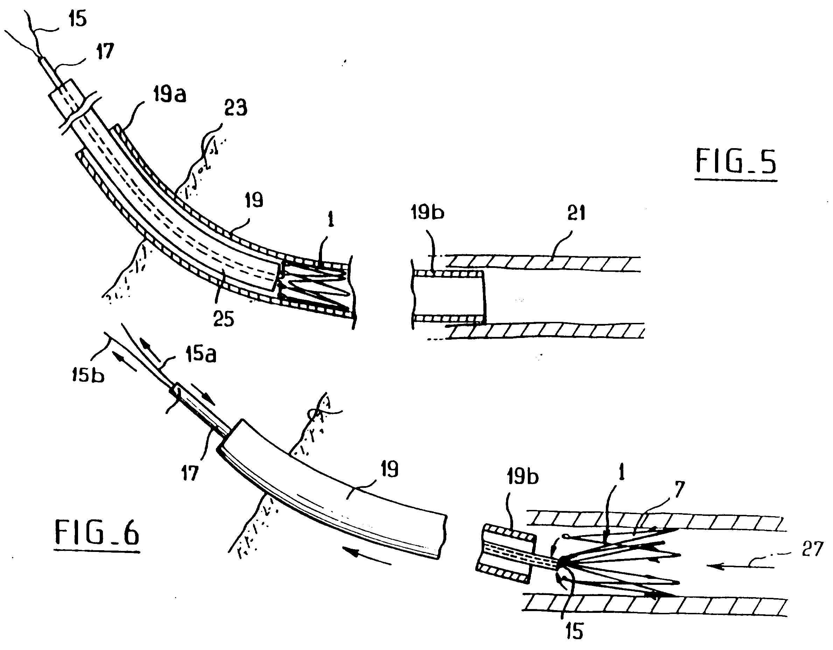

En référence aux figures 5 à 8, on va maintenant présenter brièvement une possible méthode d'implantation et d'utilisation du dispositif 1, l'implantation se faisant toujours sous anesthésie au moins locale.

Après avoir introduit, à travers la voie d'accès ménagée (veine jugulaire puis veine cave supérieure et enfin veine cave inférieure), un fin câble de guidage métallique, l'opérateur emmanche sur l'extrémité proximale du câble (qui ressort alors de la veine jugulaire), un ensemble formé d'un mandrin assez rigide et d'une gaine extérieure 19 en matériau bio-compatible.

Une fois la gaine et le mandrin parvenus jusqu'à la zone d'implantation retenue dans le vaisseau 21, le câble et le mandrin pourront être retirés.

Ainsi positionnée, la gaine 19 va servir de guidage pour la mise en place du dispositif 1 qui est alors habituellement préconditionné à l'état radialement replié de ses pattes.

Pour parvenir jusqu'à l'extrémité distale 19b de la gaine, le dispositif 1 (entraînant derrière lui son fil bouclé 15 passant dans le fin cathéter de commande 17) est poussé par un poussoir creux 25, à travers lequel passe le fin tube 17 (figure 5).

Une fois le dispositif 1 parvenu à l'extrémité 19b de la gaine, il va alors naturellement s'expanser élastiquement en s'appliquant contre la paroi intérieure de la veine, avec fixation via les crochets 11.

L'opérateur pourra alors retirer la gaine 19 et si nécessaire tirer sur les extrémités 15a, 15b du fil 15 pour resserrer les pattes 7.

Pour éviter de laisser l'extrémité proximale 17a du tube 17 ressortir de la peau du patient, l'opérateur peut alors réserver un petit logement sous-cutané 29 où il peut placer cette extrémité du tube après en avoir adapté la longueur et avoir fixé l'extrémité débouchante du fil 15, par exemple par un noeud 31, maintenant en position rapprochée de filtration les pattes concernées du dispositif. Pour le repérage, il pourra sertir autour du cathéter une pièce ovoïde en forme d'olive 32.

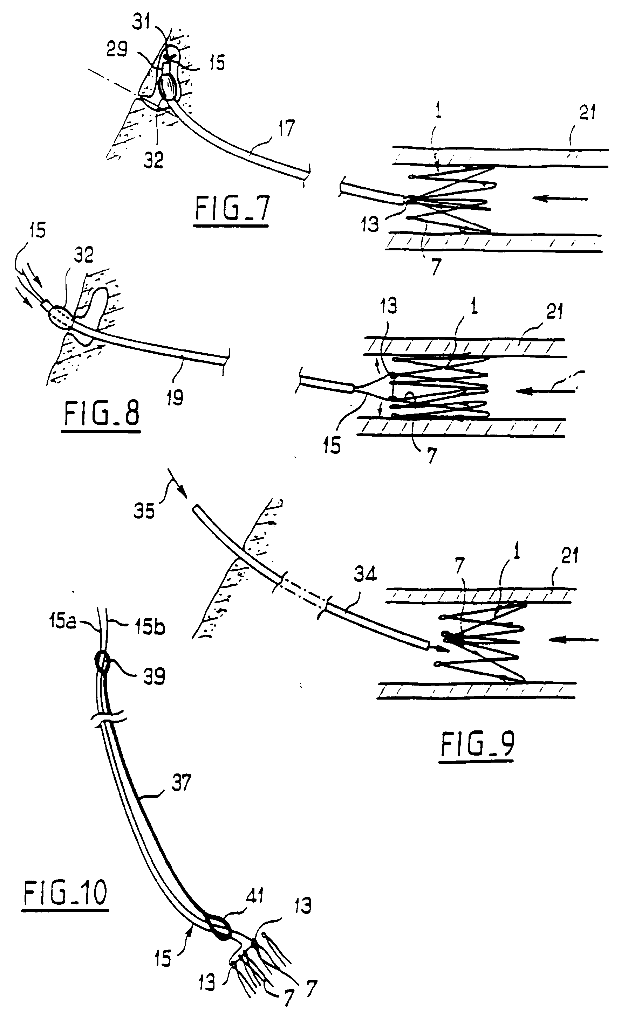

Une fois tout ceci enfoui dans le logement, l'opérateur refermera la voie d'accès de manière à emprisonner le tout sous la peau, après suture, comme illustré sur la figure 7.

Le filtre peut par exemple être laissé ainsi pendant une quinzaine de jours, voire un mois et plus, jusqu'à ce que le risque cardio-vasculaire qui a nécessité sa mise en place soit éliminé.

A ce moment, il va suffire au praticien de ressortir "l'olive" 32 de son logement, de retirer la fixation du fil 15 et laisser filer ce dernier pour relâcher la contrainte exercée sur les pattes, lesquelles vont alors naturellement se déployer en ouvrant totalement le dispositif, qui reprend alors sa forme naturelle de la figure 2.

Le sang peut alors passer librement à travers le dispositif qui ne joue plus son rôle de filtre.

Si le praticien le juge alors utile (et notamment dans l'éventualité où il aurait encore besoin ultérieurement de redonner au dispositif une capacité de filtration), il pourra simplement replacer dans son logement l'olive 32 et le fil 15, et refermer, comme sur la figure 7, la voie d'accès par une suture.

Bien entendu, différentes variantes de réalisation du dispositif de l'invention pourrait être imaginées.

Ainsi, tout d'abord, tous les sommets des pattes 7 pourraient être réunis ensemble à une même extrémité, le fil passant alors dans autant d'oeillets 13.

On pourraît également prévoir de réaliser une patte en "V" sur deux dans un alliage à mémoire de forme, par exemple à mémoire de forme thermique, comme le "Nitinol" (marque déposée) alliage à base d'environ 50 % de Titane et 50 % de Nickel.

La sollicitation de l'alliage à mémoire retenue, pourraît être obtenue dans le cadre d'une sollicitation thermique, au moyen d'un cathéter 34 venant depuis l'extérieur, apporter au niveau du "cône de filtration" (qui serait l'état dans lequel le dispositif serait implanté), un fluide chauffant ou refroidissant 35 (tel que du sérum physiologique) assurant une ouverture du cône et une libération consécutive du vaisseau (voir figure 9).

Une autre solution pour passer de la position filtrante à la position non filtrante, et inversement, une fois le dispositif implanté, serait d'utiliser un long câble relativement flexible 37 terminé à chaque extrémité par un oeillet 39, 41, les deux brins 15a, 15b du fil bouclé 15 passant à travers les deux oeillets ainsi qu'à travers ceux 13 de certaines au moins des pattes 7, de telle manière à pouvoir, depuis au-delà de l'oeillet 39, assurer à distance une traction avec resserrement, ou un relâchement avec écartement, desdites pattes (voir figure 10).

En variante, on aurait pu prévoir que les lignes des zigzags des pattes ne soient pas rectilignes, mais par exemple conformées pour être sinueuses, et ce, en utilisant plusieurs fils métalliques.

Un prothèse présentant intérieurement une forme de sablier en position de filtration (avec deux zones coniques tête bêche) aurait également pu être imaginée.

Ainsi donc, ce n'est pas tant la forme en zigzag des pattes qui paraît a priori le coeur de l'invention, que la possibilité d'obtenir en permanence, lorsque la prothèse est implantée, d'une part une surface ouverte de paroi tubulaire qui stabilise efficacement cette prothèse dans l'axe du vaisseau avec, d'autre part une possibilité de déformation locale de cette surface ouverte pour constituer une zone de striction à géométrie variable, avec passage du sang entre lesdites "ouvertures" de la surface, seule les caillots étant retenus.

## Revendications

1. Dispositif propre à être implanté jusqu'à l'intérieur d'un vaisseau du corps d'un patient, et comprenant :
- une structure comprenant une série de pattes (7) allongées essentiellement sensiblement parallèlement à un axe (3) pour définir, suivant cet axe, une surface tubulaire ouverte à travers laquelle le sang peut passer, cette structure présentant un premier diamètre de tube réduit, pour son implantation jusqu'au vaisseau, et un second diamètre de tube expansé, pour être propre à venir au contact de ce vaisseau dans une position implantée à l'intérieur de celui-ci,
- et des moyens (15, 17) de sollicitation des pattes (7) de la structure, alors que celle-ci présente son second diamètre expansé de tube, pour créer une zone de striction,
**caractérise en ce que,** pour constituer un filtre sanguin sélectif, lesdits moyens de sollicitation (15, 17) sont reliés à certaines seulement des pattes (7) afin
soit de rapprocher de l'axe (3) les pattes sollicitées, jusqu'à une position de filtration appropriée à la retenue de caillots pouvant être contenus dans le sang passant à travers la structure,
soit, à partir de cette position de filtration, d'écarter radialement dudit axe ces mêmes pattes (7), en faisant alors varier la capacité de filtration du dispositif.

2. Dispositif selon la revendication 1, **caractérisé en ce que** :
- la structure présente une série de lignes reliées par des portions d'extrémité courbées, pour définir lesdites pattes (7), suivant une configuration en zigzags enroulée selon ladite surface tubulaire ouverte,
- et les moyens de sollicitation (15, 17) sont reliés à certaines seulement desdites portions d'extrémité courbées, à l'une des extrémités de la structure.

3. Dispositif selon l'une quelconque des revendications précédentes, **caractérisé en ce que** les pattes (7) non reliées auxdits moyens de sollicitation portent, seules, des moyens d'accrochage (11), pour un accrochage du dispositif à la paroi du vaisseau (21) et une implantation définitive de celui-ci.

4. Dispositif selon l'une des revendications précédentes, **caractérisé en ce que** les pattes (7) reliées aux moyens de sollicitation (15, 17) sont réalisées en un matériau à mémoire de forme, et ce dispositif comprend en outre des moyens (34) d'amenée depuis l'extérieur du corps du patient et, jusqu'à ces pattes, d'un produit (35) déclenchant faisant évoluer en conséquence la forme de cette partie de structure.

## Claims

1. A device adapted to be implanted inside a vessel of a patient's body and comprising :
- an structure comprising a series of legs (7) which extend globally essentially parallel to an axis (3) for defining, along said axis, an open tubular surface through which the blood can pass, said structure having a first, small tube diameter, for being implanted up to the vessel, and a second expanded tube diameter so as to be adapted for contacting the vessel in an implanted position within said vessel ;
- and means (15, 17) for stressing the legs (7) of the structure, when the structure is in its second expanded tube diameter, for creating a constriction zone ; characterized in that, for obtaining a selective blood filter, said stressing means (15, 17) are connected to few than all the legs (7), for :
- either bringing the stressed legs closer to the axis (3) up to a filtering position adapted for retaining clots which may be present in the blood passing through the structure ;
- or, from said filtering position, moving said legs (7) away from said axis, such that the filtering capacity of the device is varied.

2. The device according to claim 1, characterised in that :
- the structure has a series of lines connected by curved end portions, for defining said legs (7), according to a zig-zag configuration which is wound up along said open tubular surface ;
- and the stressing means (15, 17) are connected to fewer than all the curved end portions, at one of the ends of the structure.

3. The device according to anyone of the preceding claims characterised in that only those of the legs (7) which are not connected to the stressing means have attachment means (11) for attaching the device to the wall of the vessel (21) and obtaining a definitive implantation of said device.

4. The device according to anyone of the preceding claims characterised in that those of the legs (7) which are connected to the stressing means (15, 17) are made of a shape-memory material, and the device further comprises means (34) for supplying to those legs and from outside the patient's body a trigger substance (35) which consequently develops the shape of this portion of the structure.

## Patentansprüche

1. Vorrichtung zum Implantieren in das Innere eines Körpergefässes eines Patienten, bestehend aus:
- einer Struktur bestehend aus einer Reihe von Krallen (7), die im wesentlichen parallel zu einer Achse (3) ausgerichtet sind, um entlang dieser Achse eine offene, röhrenförmige Wand zu bilden, durch die das Blut fließen kann, wobei diese Struktur für seine Einpflanzung bis zum Gefäß einen ersten verminderten Röhrendurchmesser aufweist und einen zweiten, vergrößerten Röhrendurchmesser, um in einer eingepflanzten Position in dem Gefäß zur Anlage an diesem zu kommen,
- und Mitteln (15, 17) um die Krallen (7) der Struktur zu belasten, wenn sich diese in dem zweiten, vergrößerten Röhrendurchmesser darstellt, um eine Einschnürung zu schaffen,
**dadurch gekennzeichnet, daß,** um einen selektiven Blutfilter zu bilden, die Belastungsmittel (15, 17) nur mit einigen der Krallen (7) verbunden sind, um
entweder die belasteten Krallen bis zu einer geeigneten Filterposition an die Achse (3) anzunähern, damit Blutgerinnsel, die in dem die Struktur durchfließenden Blut enthalten sein können, zurückgehalten werden,
oder die Krallen (7) nach der Filterposition radial aufzuspreizen, um die Filterkapazität der Vorrichtung zu verändern.

2. Vorrichtung nach Anspruch 1, **dadurch gekennzeichnet, daß**:
- die Struktur aus Linien besteht, die durch gekrümmte Endteile miteinander verbunden sind um die Krallen (7) als eingerolltes Zickzack-förmiges Gebilde entsprechend der offenen röhrenförmigen Wand zu definieren,
- und die Belastungsmittel (15, 17) nur mit einigen der gekrümmten Endteile an einem Ende der Struktur verbunden sind.

3. Vorrichtung nach einem der vorhergehenden Ansprüche, **dadurch gekennzeichnet, daß** nur die Krallen (7) die nicht mit den Belastungsmitteln verbunden sind Mittel (11) zum Verhaken tragen, um die Vorrichtung mit der Gefäßwand (21) zu verhaken und definitiv dort zu implantieren.

4. Vorrichtung nach einem der vorhergehenden Ansprüche, **dadurch gekennzeichnet, daß** die Krallen (7), die mit den Belastungsmitteln (15, 17) verbunden sind, aus einem formspeichernden Material bestehen und diese Vorrichtung außerdem Zuführungsmittel (34) von außerhalb des Körpers des Patienten bis zu den Krallen für ein auslösendes Produkt (35) aufweist, durch das sich die Form dieses Teils der Struktur verändern läßt.
